(19) Europäisches Patentamt
European Patent Office
Office européen des brevets

(11) EP 4 717 694 A1

(12) **EUROPEAN PATENT APPLICATION**
published in accordance with Art. 153(4) EPC

(43) Date of publication:
**01.04.2026 Bulletin 2026/14**

(21) Application number: **23937865.6**

(22) Date of filing: **22.05.2023**

(51) International Patent Classification (IPC):
***C07D 403/06*** *(2006.01)*

(52) Cooperative Patent Classification (CPC):
**C07D 403/06**

(86) International application number:
**PCT/CN2023/095443**

(87) International publication number:
**WO 2024/239186 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Iregene Therapeutics Co., Ltd.**
**Chengdu, Sichuan 610200 (CN)**

(72) Inventors:
- **WEI, Jun**
  **Suzhou, Jiangsu 215021 (CN)**
- **CHEN, Xiping**
  **Suzhou, Jiangsu 215021 (CN)**
- **ZHU, Yasha**
  **Suzhou, Jiangsu 215021 (CN)**
- **YAN, Ruorong**
  **Suzhou, Jiangsu 215021 (CN)**
- **CAI, Meng**
  **Suzhou, Jiangsu 215021 (CN)**

(74) Representative: **Metida**
**Gyneju str. 16**
**01109 Vilnius (LT)**

# (54) PYRAZOLFORMYL PIPERAZINONE COMPOUND, PHARMACEUTICAL COMPOSITION AND USE THEREOF

(57) The present application relates to the technical field of medicines, in particular to a pyrazolformyl piperazinone compound, a pharmaceutical composition and the use thereof. The pyrazolformyl piperazinone compound has a structure as shown in formula I, or is a pharmaceutically acceptable salt, ester, amide, solvate, active metabolite, polymorph, isotopic label, isomer or prodrug of the structure as shown in formula I.

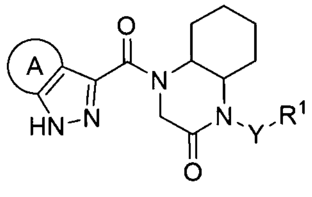

I

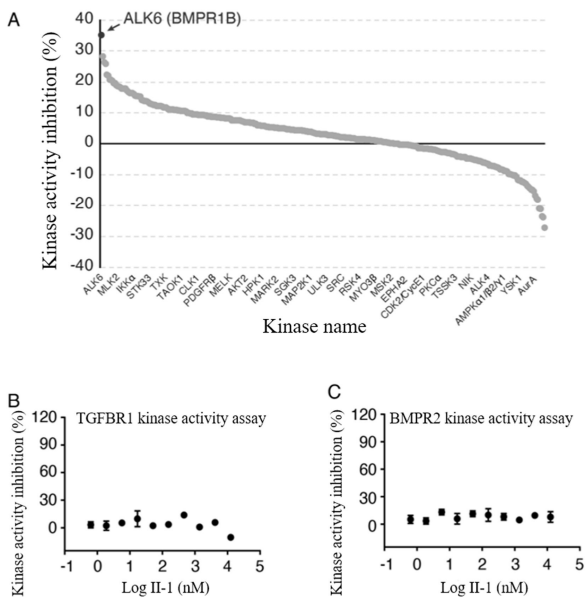


FIG. 1

EP 4 717 694 A1

## Description

BACKGROUND OF THE INVENTION

Field of the Invention

The present application relates to the field of pharmaceutical technology, and in particular to pyrazolecarbonyl piperazinone compounds, pharmaceutical compositions thereof, and uses thereof.

Description of Related Art

Transforming growth factor-β (TGF-β) belongs to the TGF-β superfamily and plays a critical role in regulating cell growth and differentiation. Dysfunction of the TGF-β signaling pathway is associated with human diseases, including cancer, fibrosis, systemic sclerosis, and genetic disorders (Wakefield LM and Hill CS. Nat Rev Cancer 2013, 13: 328-341). For example, in advanced cancers, TGF-β often promotes tumor progression and metastasis, acting as a tumorigenic factor (Massagué. J. Cell. 2008, 134: 215-230).

The cell membrane receptors of TGF-β are mainly divided into two types: type I TGF-β receptors and type II TGF-β receptors. In vertebrates, seven type I receptors (Activin-receptor like kinases, ALKs) are known: ALK1 (ACVRL1), ALK2 (ACVR1), ALK3 (BMPR1A), ALK4 (ACVR1B), ALK5 (TGFBR1), ALK6 (BMPR1B), and ALK7 (ACVR1C); and five type II receptors: TGFβRII, ACTRII, ACTRII B, BMPRII, and AMHRII. Among them, the ALK6 receptor belonging to type I, i.e., BMPR1B (bone morphogenetic protein receptor, type IB), has been found to be associated with multiple prevalent diseases, such as breast cancer, gastric cancer, and pulmonary arterial hypertension. Therefore, strategies to deliberately inhibit BMPR 1B for the treatment of related diseases represent a pressing and significant challenge in the pharmaceutical field.

BRIEF SUMMARY OF THE INVENTION

Based on the above, it is necessary to provide a pyrazolecarbonyl piperazinone compound capable of selectively inhibiting BMPR1B, exhibiting antitumor activity, and having potential for clinical application in the treatment of cancer.

In a first aspect, the present application provides a pyrazolecarbonyl piperazinone compound, the pyrazolecarbonyl piperazinone compound has a structure represented by Formula I, or a pharmaceutically acceptable salt, ester, amide, solvate, active metabolite, polymorph, isotopically labeled compound, isomer, or prodrug thereof;

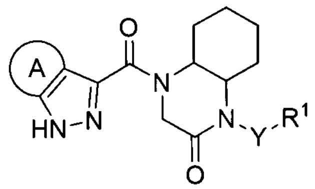

I

wherein the ring A is selected from any one of the following structures:

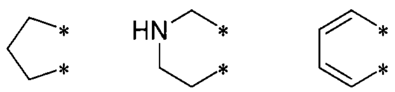

Y is $-(CH_2)_n-$, where n is 0, 1, 2, or 3;
$R^1$ is selected from -H, -D, unsubstituted or $R^2$-substituted $C_1$-$C_6$ alkyl, $C_1$-$C_6$ alkoxy, $C_6$-$C_{10}$ aryl, or $C_3$-$C_{10}$ heteroaryl;
$R^2$ is selected from -F, -Cl, -Br, -I, -OH, -COOH, $C_1$-$C_6$ alkyl, or $C_1$-$C_6$ alkoxy;
"*" represents a point of attachment.

In a second aspect, the present application provides a pharmaceutical composition comprising the aforementioned pyrazolecarbonyl piperazinone compound and at least one pharmaceutically acceptable carrier.

In a third aspect, the present application provides the use of the aforementioned pyrazolecarbonyl piperazinone compound or the aforementioned pharmaceutical composition in the preparation of a medicament for treating one or more of the following diseases: cancer and pulmonary arterial hypertension.

In a fourth aspect, the present application provides the use of the aforementioned pyrazolecarbonyl piperazinone compound as a BMPR1B inhibitor.
In a fifth aspect, the present application provides a method for inhibiting BMPR1B expression, comprising the step of contacting the aforementioned pyrazolecarbonyl piperazinone compound with cells, biological tissues, or organoids comprising BMPR1B.
In a sixth aspect, the present application provides a method for treating cancer or pulmonary arterial hypertension, comprising the step of administering to a subject a therapeutically effective amount of the aforementioned pyrazolecarbonyl piperazinone compound, or administering to a subject a therapeutically effective amount of the aforementioned pharmaceutical composition.

BRIEF DESCRIPTION OF THE SEVERAL VIEWS OF THE DRA WING(S)

Figure 1 shows the specificity of the binding between pyrazolecarbonyl piperazinone compounds and their targets based on kinase activity screening analysis. Figure 1A presents the effect of compound II-1 on the enzymatic activity of 330 kinases. Figures 1B and 1C show the kinase activity assay results of compound II-1 against TGFBR1 and BMPR2 at different concentrations.
Figure 2 shows the interaction between the pyrazolecarbonyl piperazinone compound and BMPR1B in the pyrazolecarbonyl piperazinone-BMPR1B complex, by using PyMol software. In Figure 2A, the compound is II-13; in Figure 2B, the compound is II-11; in Figure 2C, the compound is II-2; and in Figure 2D, the compound is II-1.
Figure 3 shows the cytotoxicity assay results of compound II-1 and LY2157299 on tumor cells.

DETAILED DESCRIPTION OF THE INVENTION

For the purpose of facilitating the understanding of the present application, the following provides a more detailed description of the present application with reference to the accompanying drawings. The drawings illustrate preferred embodiments of the present application. However, the present application may be implemented in many different forms and is not limited to the embodiments described herein. Rather, the purpose of providing these embodiments is to enable a more thorough and comprehensive understanding of the disclosure of the present application.
Unless otherwise defined, all technical and scientific terms used herein have the same meanings as those commonly understood by persons skilled in the art to which the present application pertains. The terminology used in the specification of the present application is solely for the purpose of describing particular embodiments and is not intended to limit the present application. The term "and/or" as used herein includes any and all combinations of one or more of the listed items.
In the present application, an open-ended description of technical features includes both a closed-ended technical solution composed of the listed features and an open-ended technical solution comprising the listed features.
In the present application, numerical ranges are considered continuous and include the minimum and maximum values of the range, as well as any value therebetween, unless otherwise specified. Furthermore, when the range refers to integers, it includes every integer between the minimum and maximum values. In addition, when multiple ranges are described for a feature or property, the ranges may be combined. In other words, unless otherwise indicated, all ranges disclosed herein are understood to include any and all sub-ranges therein.
Percentages disclosed in the present application, unless otherwise specified, refer to weight percent for solid-liquid and solid-solid mixtures, and volume percent for liquid-liquid mixtures.
Percentage concentrations disclosed herein, unless otherwise specified, refer to the final concentration, which is the proportion of the added component in the system after addition.
Temperature parameters disclosed herein, unless otherwise specified, allow for both constant-temperature treatment and treatment within a certain temperature range. Constant-temperature treatment permits fluctuations within the precision range of the instrument.

Terminology Definitions

The term "alkyl" refers to a monovalent residue derived from a saturated hydrocarbon by removal of a hydrogen atom, wherein the carbon atom may be primary, secondary, tertiary, quaternary, or a combination thereof. Phrases containing this term, such as "$C_1$-$C_6$ alkyl," refer to an alkyl group containing 1 to 6 carbon atoms, and each occurrence may independently be a $C_1$, $C_2$, $C_3$, $C_4$, $C_5$, or $C_6$ alkyl. Suitable examples include, but are not limited to: methyl (Me, $-CH_3$), ethyl (Et, $-CH_2CH_3$), 1-propyl (n-Pr, $-CH_2CH_2CH_3$), 2-propyl (i-Pr, $-CH(CH_3)_2$), 1-butyl (n-Bu, $-CH_2CH_2CH_2CH_3$), 2-methyl-1-propyl (i-Bu, $-CH_2CH(CH_3)_2$), 2-butyl (s-Bu, $-CH(CH_3)CH_2CH_3$), 2-methyl-2-propyl (t-Bu, $-C(CH_3)_3$), 1-pentyl (n-pentyl,

$-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), 1-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), and 3,3-dimethyl-2-butyl ($-CH(CH_3)C(CH_3)_3$.

The term "alkoxy" refers to a group of the structure -O-alkyl, i.e., an alkyl group as defined above linked via an oxygen atom to an adjacent moiety. Phrases containing this term, such as "$C_1$-$C_6$ alkoxy," refer to an alkyl portion containing 1-6 carbon atoms, and each occurrence may independently be $C_1$, $C_4$, $C_5$, or $C_6$ alkoxy. Suitable examples include, but are not limited to: methoxy ($-O-CH_3$ or -OMe), ethoxy ($-O-CH_2CH_3$ or - OEt), and tert-butoxy ($-O-C(CH_3)_3$ or -OtBu).

The term "aryl" refers to an aromatic hydrocarbon group derived from an aromatic ring compound by removal of a hydrogen atom and may be monocyclic, fused, or polycyclic, with at least one aromatic ring in the system for polycyclic structures. For example, "$C_6$-$C_{10}$ aryl" refers to an aryl group containing 6-10 carbon atoms, each occurrence independently being $C_6$, $C_7$, $C_8$, $C_9$, or $C_{10}$ aryl. Suitable examples include, but are not limited to: phenyl, biphenyl, naphthyl, and their derivatives.

The term "heteroaryl" refers to an aryl or cyclopentadienyl group in which at least one carbon atom is replaced by a non-carbon atom such as N, O, or S. For example, "$C_3$-$C_{10}$ heteroaryl" refers to a heteroaryl group containing 3-10 carbon atoms, each occurrence independently being $C_3$, $C_4$, $C_5$, $C_6$, $C_7$, or $C_8$ heteroaryl. Suitable examples include, but are not limited to: furan, benzofuran, thiophene, benzothiophene, pyrrole, pyrazole, triazole, imidazole, oxazole, isoxazole, thiazole, tetrazole, indole, carbazole, pyrrolo[1,2-a]imidazole, pyrrolo[1,2-a]pyrrole, thieno[2,3-b]pyrrole, thieno[3,2-b]thiophene, furano[2,3-b]pyrrole, furano[2,3-b]furan, thieno[2,3-b]furan, benzoisoxazole, benzoisothiazole, benzimidazole, pyridine, pyrazine, diazine, pyrimidine, triazine, quinoline, isoquinoline, naphthyridine, quinoxaline, phthalazine, quinazoline, and quinazolinone.

The term "prodrug" refers to any compound that, upon administration to a biological system, undergoes spontaneous chemical reaction, enzymatic transformation, photolysis, and/or metabolic chemical reaction to produce the active drug, i.e., the active ingredient. Prodrugs are thus covalently modified analogs or latent forms of therapeutically active compounds. Suitable examples include, but are not limited to: carboxylic esters, carbonates, phosphate esters, nitrate esters, sulfate esters, sulfonates, sulfamates, amino compounds, carbamates, azo compounds, phosphoramides, glycosides, ethers, acetal derivatives, and the like.

The term "pharmaceutically acceptable" refers to ligands, materials, compositions, and/or dosage forms that are suitable for administration to a patient within the bounds of reasonable medical judgment and having a reasonable benefit/risk ratio.

The term "pharmaceutically acceptable carrier" refers to pharmaceutically acceptable materials, compositions, or vehicles, such as liquid or solid fillers, diluents, excipients, solvents, or encapsulating materials. As used herein, the term "pharmaceutically acceptable carrier" includes buffers, sterile water for injection, solvents, dispersing media, coatings, antibacterial and antifungal agents, isotonic agents, absorption retardants, and the like, which are compatible with the administration of the drug. Each carrier must be "pharmaceutically acceptable" in the sense of being compatible with other ingredients of the formulation and non-toxic to the patient. Suitable examples include, but are not limited to: (1) sugars such as lactose, glucose, and sucrose; (2) starches such as corn starch, potato starch, and substituted or unsubstituted β-cyclodextrins; (3) cellulose and derivatives such as sodium carboxymethylcellulose, ethylcellulose, and cellulose acetate; (4) powdered tragacanth; (5) malt; (6) gelatin; (7) talc; (8) excipients such as cocoa butter and suppository waxes; (9) oils such as peanut oil, cottonseed oil, safflower oil, sesame oil, olive oil, corn oil, and soybean oil; (10) glycols such as propylene glycol; (11) polyols such as glycerol, sorbitol, mannitol, and polyethylene glycol; (12) esters such as ethyl oleate and ethyl laurate; (13) agar; (14) buffers such as magnesium hydroxide and aluminum hydroxide; (15) alginates; (16) pyrogen-free water; (17) isotonic saline; (18) Ringer's solution; (19) ethanol; (20) phosphate-buffered saline; and (21) other non-toxic compatible substances used in pharmaceutical formulations.

The term "pharmaceutically acceptable salt" refers to any salt of the compounds disclosed herein with acids or bases suitable for use as a medicament. Pharmaceutically acceptable salts include inorganic and organic salts. One type of salt is a salt of the disclosed compound with an acid. Suitable acids include, but are not limited to: inorganic acids such as hydrochloric acid, hydrobromic acid, hydrofluoric acid, sulfuric acid, nitric acid, and phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, benzoic acid, methanesulfonic acid, ethanesulfonic acid, p-toluenesulfonic acid, benzenesulfonic acid, naphthalenesulfonic acid; and amino acids such as proline, phenylalanine, aspartic acid, glutamic acid. Another type of salt is a salt of the disclosed compound with a base. Suitable bases include, but are not limited to: alkali metal salts (e.g., sodium or potassium), alkaline earth metal salts (e.g., magnesium or calcium), ammonium salts (e.g., lower alkanol ammonium salts and other pharmaceutically acceptable amine salts) such as methylamine, ethylamine, propylamine, dimethylamine, trimethylamine, diethylamine, triethylamine, tert-butylamine,

ethylenediamine, hydroxyethylamine, dihydroxyethylamine, tri(hydroxyethyl)amine, and amine salts derived from morpholine, piperazine, or lysine.

The term "pharmaceutically acceptable ester or amide" refers to any ester or amide of the disclosed compounds suitable for use as a medicament. Pharmaceutically acceptable esters include organic and inorganic esters.

In cases where the compound of Formula I contains a carboxyl group, pharmaceutically acceptable esters or amides can be prepared by conventional methods. For example, the compound of Formula I or its active derivative (e.g., an acyl chloride, mixed anhydride, or the like) can be reacted with a suitable alcohol (e.g., a $C_1$-$C_6$ alcohol) or its active derivative (e.g., (5-methyl-2-oxo-1,3-dioxolen-4-yl)methyl, neopentyl, benzofuranone, [(isopropoxycarbonyl)oxy]methyl, or the like), or with ammonia or a suitable amine (e.g., a mono-$C_1$-$C_6$ alkylamine or a di-$C_1$-$C_6$ alkylamine). Similarly, the ester of the compound of Formula I can be reacted with a suitable alcohol (e.g., a $C_1$-$C_6$ alcohol) via transesterification, or with ammonia or a suitable amine (e.g., a mono-$C_1$-$C_6$ alkylamine or a di-$C_1$-$C_6$ alkylamine) to form an amide. Alternatively, the alkali metal salt of the compound of Formula I can be reacted with an appropriate halide (e.g., a $C_1$-$C_6$ alkyl chloride or bromide, (5-methyl-2-oxo-1,3-dioxolen-4-yl)methyl chloride or bromide, neopentyl chloride or bromide, benzofuranone chloride or bromide, [(isopropoxycarbonyl)oxy]methyl chloride or bromide, or the like) to produce pharmaceutically acceptable esters or amides. In a similar manner, when the compound of Formula I contains a hydroxyl group, pharmaceutically acceptable esters can be prepared by conventional condensation reactions with carboxylic acids, acyl chlorides, acid anhydrides, or their active derivatives.

The aforementioned esters may include, for example, $C_1$-$C_6$ alkyl esters such as methyl, ethyl, propyl, isopropyl, butyl, sec-butyl, tert-butyl, pentyl, and hexyl esters; $C_3$-$C_6$ cycloalkyl esters such as cyclopentyl and cyclohexyl esters; $C_6$-$C_{10}$ aryl esters such as phenyl and naphthyl esters; $C_6$-$C_{10}$ aryl-$C_1$-$C_6$ alkyl esters such as benzyl, phenethyl, $\alpha$-methylbenzyl, 3-phenylpropyl, 4-phenylbutyl, 6-phenylhexyl, diphenylmethyl, and triphenylmethyl esters; or hydrolyzable esters in vivo such as (5-methyl-2-oxo-1,3-dioxolen-4-yl)methyl ester, neopentyl ester, benzofuranone ester, [(isopropoxycarbonyl)oxy]methyl ester, [(cyclohexyloxycarbonyl)oxy]methyl ester, and 1-[(cyclohexyloxycarbonyl)oxy]ethyl ester.

The aforementioned amides may include, for example, primary amides ($-CONH_2$); mono-$C_1$-$C_6$ alkyl or mono-$C_3$-$C_6$ cycloalkyl amides such as N-methylamide, N-ethylamide, N-propylamide, N-isopropylamide, N-butylamide, N-sec-butylamide, N-tert-butylamide, N-pentylamide, N-hexylamide, N-cyclopropylamide, N-cyclopentylamide, and N-cyclohexylamide; or di-$C_1$-$C_6$ alkyl, N-$C_1$-$C_6$ alkyl-N-$C_3$-$C_6$ cycloalkyl, or di-$C_3$-$C_6$ cycloalkyl amides such as N,N-dimethylamide, N,N-diethylamide, N,N-dipropylamide, N,N-diisopropylamide, N-methyl-N-ethylamide, N-methyl-N-propylamide, N-methyl-N-butylamide, N-ethyl-N-propylamide, N-ethyl-N-butylamide, N-butyl-N-cyclopentylamide, N-ethyl-N-cyclopropylamide, and N,N-dicyclohexylamide.

The term "solvate" refers to a complex formed between a compound of Formula I and solvent molecules in a defined stoichiometric ratio. The term "hydrate" refers to a complex formed between the compound of the present application and water.

The term "active metabolite" refers to a derivative of the compound that exhibits pharmacological activity upon metabolism of the compound.

The term "polymorph" refers to the compound of the present application existing in different crystalline lattice forms.

The term "isotopically labeled compound" refers to a compound of the present application labeled with one or more isotopes. Isotopes may include, for example, isotopes of H, C, N, O, P, F, and S, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}S$.

The term "isomer" refers to compounds having the same molecular formula but differing in the spatial arrangement of atoms. Compounds of the present application may contain asymmetric or chiral centers, double bonds, or other structural elements and may thus include optical isomers, geometric isomers, tautomers, atropisomers, and other stereoisomeric forms. These isomers, including individual stereoisomers and racemates, are encompassed within the scope of the present application. For example, optical isomers can be prepared using chiral resolution, chiral synthesis, chiral reagents, or other conventional techniques to obtain optically active (R)- and (S)-enantiomers, as well as D and L forms. Non-racemic isomers can also be generated by reaction with a suitable chiral reagent (e.g., a chiral alcohol or Mosher's acid chloride), separated, and converted (e.g., by hydrolysis) to the corresponding single stereoisomer. Separation can also be achieved via chromatographic methods.

"Pharmaceutical compositions" may be prepared by methods well known in the art and can be administered by various routes depending on the need for local or systemic treatment and the target area.

## Modes of Administration

The dosage forms and modes of administration of the compounds of the present application or pharmaceutical compositions thereof are not particularly limited.

Representative modes of administration include, but are not limited to, oral, intratumoral, rectal, parenteral

(intravenous, intramuscular, or subcutaneous) injection, and topical administration.

Solid dosage forms for oral administration include capsules, tablets, pills, powders, and granules. In these solid dosage forms, the active compound can be mixed with at least one conventional inert excipient (or carrier), such as sodium citrate or dicalcium phosphate, or with one or more of the following: (a) fillers or bulking agents, e.g., starch, lactose, sucrose, glucose, mannitol, and silicates; (b) binders, e.g., hydroxymethylcellulose, alginates, gelatin, polyvinylpyrrolidone, sucrose, and gum arabic; (c) humectants, e.g., glycerin; (d) disintegrants, e.g., agar, calcium carbonate, potato starch, tapioca starch, alginate, certain composite silicates, and sodium carbonate; (e) retardants, e.g., paraffin; (f) absorption accelerators, e.g., quaternary ammonium compounds; (g) wetting agents, e.g., cetyl alcohol and glyceryl monostearate; (h) adsorbents, e.g., kaolin; and (i) lubricants, e.g., talc, calcium stearate, magnesium stearate, solid polyethylene glycol, sodium lauryl sulfate, or mixtures thereof. Capsules, tablets, and pills may also contain buffering agents. Solid dosage forms such as tablets, sugar-coated pills, capsules, pills, and granules may be prepared with coatings or shells, e.g., enteric coatings or other materials known in the art. Such coatings may include opacifying agents and may allow delayed release of the active compound in a specific region of the gastrointestinal tract. Exemplary encapsulating components include polymers and waxes. If necessary, the active compound may also be formulated as microcapsules with one or more of the excipients described above.

Liquid dosage forms for oral administration include pharmaceutically acceptable emulsions, solutions, suspensions, syrups, or tinctures. In addition to the active compound, liquid dosage forms may contain inert diluents conventionally used in the art, such as water or other solvents, solubilizers, and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, sesame oil, or mixtures thereof. In addition to these inert diluents, the compositions may also contain adjuvants such as wetting agents, emulsifiers, suspending agents, sweeteners, flavoring agents, and perfumes. Suspensions may contain suspending agents, e.g., ethoxylated isostearyl alcohol, polyoxyethylene sorbitol and dehydrated sorbitol esters, microcrystalline cellulose, aluminum methoxide, agar, or mixtures thereof.

Parenteral compositions may include physiologically acceptable sterile aqueous or non-aqueous solutions, dispersions, suspensions, or emulsions, as well as sterile powders for reconstitution into sterile injectable solutions or dispersions. Suitable aqueous or non-aqueous carriers, diluents, solvents, or excipients include water, ethanol, polyols, and suitable mixtures thereof.

Topical dosage forms include ointments, powders, patches, sprays, and inhalation formulations. They may be prepared by mixing the active ingredient under sterile conditions with a pharmaceutically acceptable carrier and any preservative, buffer, or, if necessary, a propellant.

As used herein, "pharmaceutical" includes any agent, compound, composition, or mixture that provides a physiological and/or pharmacological effect in vivo or in vitro, typically producing a beneficial effect. The range of physiological and/or pharmacological effects produced by the "pharmaceutical" is not particularly limited and may be systemic or localized. The active agent in the "pharmaceutical" is not specifically limited and may be an agent capable of interacting with other substances or an inert substance that does not interact.

As used herein, a "therapeutically effective amount" refers to an amount of a compound of the present application sufficient to elicit a biological or medical response in an individual, i.e., an amount that provides a physiological and/or pharmacological beneficial effect. Such beneficial effects include, but are not limited to, reducing or inhibiting enzyme or protein activity, improving symptoms, alleviating conditions, slowing or delaying disease progression, or preventing disease.

The TGF-β superfamily is involved in many aspects of biology, including embryonic development, organogenesis, cell fate determination, immune regulation, stress responses, and stem cell functions. Accordingly, numerous studies have been conducted on TGF-β, and a number of inhibitors of the TGF-β signaling pathway have already advanced into clinical stages, such as Galunisertib (LY2157299). LY2157299 is a small-molecule inhibitor that selectively inhibits the kinase activity of TβRI. It has been investigated, either as a monotherapy or in combination with standard antitumor regimens, in patients with different cancers such as glioblastoma, pancreatic cancer, and hepatocellular carcinoma (Rodon J et al., Clin Cancer Res 2015, 21: 553-560). In the initial Phase I trials, LY2157299 demonstrated promising activity in patients with glioblastoma. On the other hand, in pancreatic cancer patients, LY2157299 showed superiority over gemcitabine (Melisi D et al., Cancer Res 2016, 76(14 Supplement): CT068-CT068). However, existing small-molecule inhibitors remain limited in their cytotoxic effects against tumor cells, and the spectrum of kinases that they can selectively inhibit is also restricted. Therefore, the development of more effective TGF-β signaling inhibitors, capable of selectively binding to a broader range of kinases, in order to contribute to the treatment of more diseases, particularly cancers, continues to be an urgent and unresolved problem in the pharmaceutical field.

In view of the foregoing background, in a first aspect, the present application provides a pyrazolecarbonyl piperazinone compound having the structure represented by Formula I, or a pharmaceutically acceptable salt, ester, amide, solvate, active metabolite, polymorph, isotopically labeled derivative, isomer, or prodrug thereof;

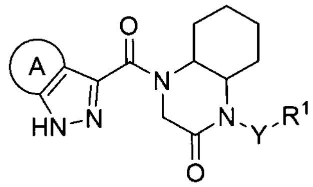

I

wherein the ring A is selected from any one of the following structures:

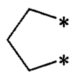 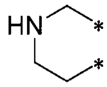 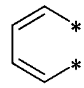

Y is $-(CH_2)_n-$, where n is 0, 1, 2, or 3;
$R^1$ is selected from -H, -D, unsubstituted or $R^2$-substituted $C_1$-$C_6$ alkyl, $C_1$-$C_6$ alkoxy, $C_6$-$C_{10}$ aryl, or $C_3$-$C_{10}$ heteroaryl;
$R^2$ is selected from -F, -Cl, -Br, -I, -OH, -COOH, $C_1$-$C_6$ alkyl, or $C_1$-$C_6$ alkoxy;
"*" represents a point of attachment.

The pyrazolecarbonyl piperazinone compounds provided in the present application, through a specially designed structure, are capable of forming relatively stable complexes with BMPR1B via hydrogen bonding, hydrophobic interactions, and the like, thereby achieving targeted binding to BMPR1B. As a result, the compounds selectively inhibit BMPR1B, effectively kill tumor cells, and prevent tumor progression and metastasis. Compared with existing small-molecule inhibitors such as LY2157299, the pyrazolecarbonyl piperazinone compounds of the present application exhibit higher cytotoxicity against tumor cells.

In some embodiments, Y is $-(CH_2)_n-$, where n is 0, 1, or 2; preferably, Y is $-(CH_2)_n-$, where n is 0 or 1; more preferably, Y is $-(CH_2)_n-$, where n is 0 or 1.

In some embodiments, $R^1$ is selected from -H, -D, unsubstituted or $R^2$-substituted $C_1$-$C_6$ alkyl, $C_6$-$C_{10}$ aryl, or $C_3$-$C_{10}$ heteroaryl. Preferably, $R^1$ is selected from -H, -D, unsubstituted or $R^2$-substituted $C_3$-$C_6$ heteroaryl. More preferably, $R^1$ is selected from -H, -D, unsubstituted or $R^2$-substituted furyl, thienyl, pyrrolyl, pyrazolyl, imidazolyl, oxazolyl, isoxazolyl, thiazolyl, isothiazolyl, pyranyl, thianyl, pyridyl, diazinyl, or pyrimidinyl. Still more preferably, $R^1$ is selected from -H, -D, or any one of the following substituents:

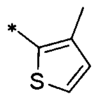 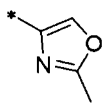 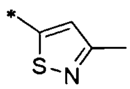 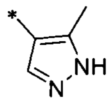 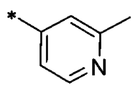

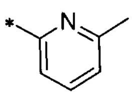 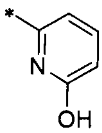 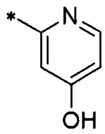 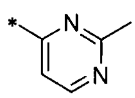 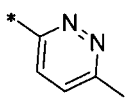

wherein "*" represents a point of attachment.

When $R^1$ is selected from heteroaryl, particularly heteroaryl substituted with a methyl or hydroxyl group, the resulting pyrazolecarbonyl piperazinone compound exhibits a higher binding energy with the docking site of BMPR1B, while showing lower physiological toxicity. This is advantageous for enhancing the efficacy and biocompatibility of the pyrazolecarbonyl piperazinone compound as a BMPR1B inhibitor, thereby providing the potential for its application in clinical treatment.

In some embodiments, $R^2$ is selected from -OH or $C_1$-$C_6$ alkyl. Preferably, $R^2$ is selected from -OH or $C_1$-$C_4$ alkyl. More preferably, $R^2$ is selected from -OH, methyl, ethyl, n-propyl, or isopropyl.

In some embodiments, the pyrazolecarbonyl piperazinone compound has a structure represented by any one of Formulas I-1 to I-13, or a pharmaceutically acceptable salt, ester, amide, solvate, active metabolite, polymorph, isotopically labeled compound, isomer, or prodrug thereof;

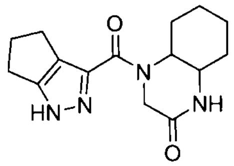

I-1

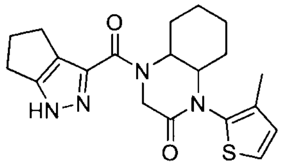

I-2

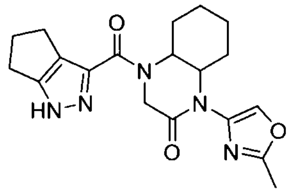

I-3

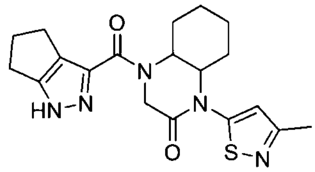

I-4

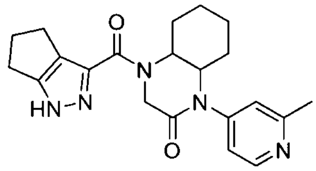

I-5

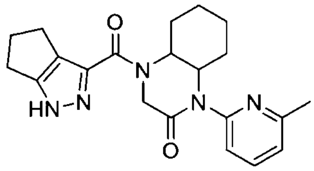

I-6

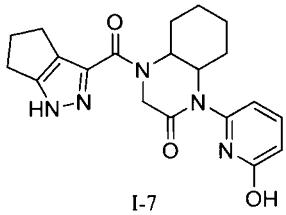

I-7

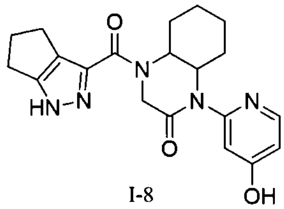

I-8

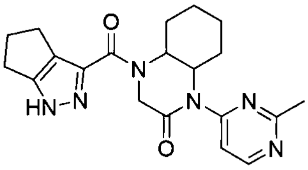

I-9

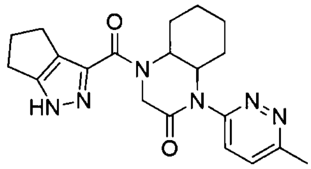

I-10

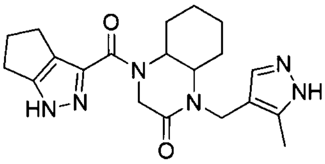

I-11

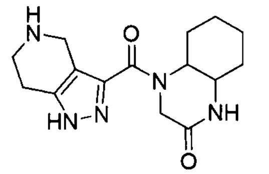

I-12

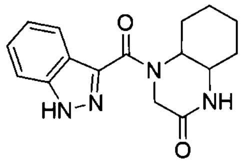

I-13

.

In some embodiments, the pyrazolecarbonyl piperazinone compound has a structure represented by Formula II, or a pharmaceutically acceptable salt, ester, amide, solvate, active metabolite, polymorph, isotopically labeled compound, isomer, or prodrug thereof;

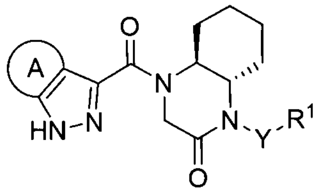

II

wherein ring A, Y, and $R^1$ are as defined in any of the foregoing embodiments.

In some embodiments, the pyrazolecarbonyl piperazinone compound has a structure represented by any one of Formulas II-1 to II-13, or a pharmaceutically acceptable salt, ester, amide, solvate, active metabolite, polymorph, isotopically labeled compound, isomer, or prodrug thereof;

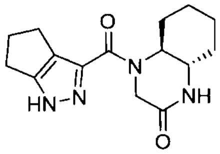

II-1

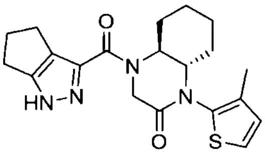

II-2

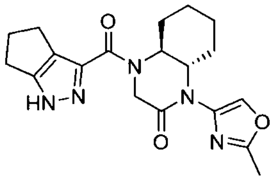

II-3

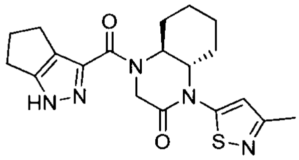

II-4

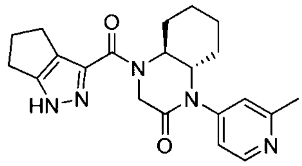
II-5

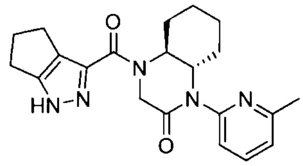
II-6

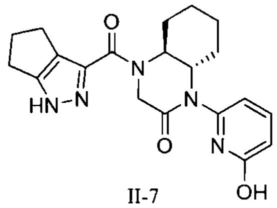
II-7

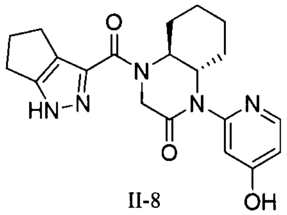
II-8

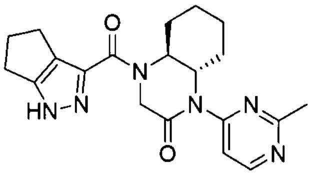
II-9

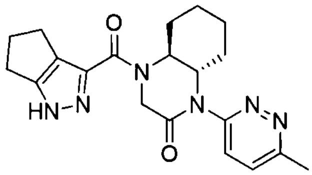
II-10

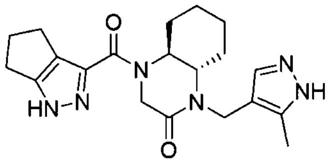
II-11

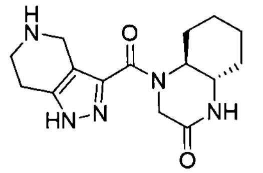
II-12

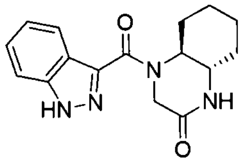
II-13

.

In a second aspect, the present application provides a pharmaceutical composition comprising the aforementioned pyrazolecarbonyl piperazinone compound and at least one pharmaceutically acceptable carrier.

In a third aspect, the present application provides the use of the aforementioned pyrazolecarbonyl piperazinone compound or the aforementioned pharmaceutical composition in the preparation of a medicament for treating one or more of the following diseases: cancer and pulmonary arterial hypertension.

In some embodiments, the cancer is one or more selected from ovarian cancer, breast cancer, glioma, and germ cell tumor.

In a fourth aspect, the present application provides the use of the aforementioned pyrazolecarbonyl piperazinone compound as a BMPR1B inhibitor.

In some embodiments, the concentration of the pyrazolylcarbonyl piperazinone compound is 10 $\mu$M to 100 $\mu$M. Optionally, the concentration of the pyrazolylcarbonyl piperazinone compound may also be, for example, 20 $\mu$M, 30 $\mu$M, 40 $\mu$M, 50 $\mu$M, 60 $\mu$M, 70 $\mu$M, 80 $\mu$M, or 90 $\mu$M.

In a fifth aspect, the present application provides a method for inhibiting BMPR1B expression, comprising the step of contacting the aforementioned pyrazolecarbonyl piperazinone compound with cells, biological tissues, or organoids comprising BMPR1B.

In some embodiments, when performing the contact treatment, the concentration of the pyrazolylcarbonyl piperazinone compound is 10 $\mu$M to 100 $\mu$M. Optionally, the concentration of the pyrazolylcarbonylpiperazinone compound may also be, for example, 20 $\mu$M, 30 $\mu$M, 40 $\mu$M, 50 $\mu$M, 60 $\mu$M, 70 $\mu$M, 80 $\mu$M, or 90 $\mu$M.

In a sixth aspect, the present application provides a method for treating cancer or pulmonary arterial hypertension, comprising the step of administering to a subject a therapeutically effective amount of the aforementioned pyrazolecarbonyl piperazinone compound, or administering to a subject a therapeutically effective amount of the aforementioned pharmaceutical composition.

In some embodiments, the cancer is one or more of ovarian cancer, breast cancer, and glioma.

Compound Synthesis:

In the following, if there is a discrepancy between the compound name and the structural formula, the structural formula shall prevail.

Synthetic Route A

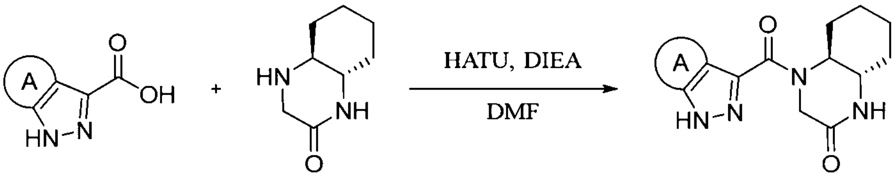


M1-n M2 II-n

Intermediate M1-n (0.12 mmol), (4aS,8aS)-tetrahydroquinoline-2(1H)-one (M2, 15 mg, 0.1 mmol), and 2-(7-azabenzotriazol)-N,N,N',N'-tetramethyluronium hexafluorophosphate (HATU, 380 mg, 1 mmol) were mixed, then N,N-diisopropylethylamine (DIEA, 2 mL) and N,N-dimethylformamide (DMF, 20 mL) were added. The reaction mixture was stirred at room temperature for 18 hours. Ethyl acetate and water were added to the reaction mixture, the organic layer was separated, dried over sodium sulfate, and concentrated under reduced pressure. The residue was purified by silica gel column chromatography (ethyl acetate : hexane = 3:7) to obtain the target product II-n.

Compounds II-1 and II-13 were obtained via Synthetic Route A.

II-1:
(4aS,8aS)-4-(1,4,5,6-tetrahydrocyclopenta[c]pyrazole-3-carbonyl)octahydroquinoxalin-2(1H)-one (12 mg, 41%), LC- MS m/z=289.2[M+H]$^+$。
II-13:
(4aS,8aS)-4-(1H-indazole-3-carbonyl)octahydroquinoxalin-2(1H)-one (17 mg, 57%), LC- MS m/z=299.2 [M+H]$^+$。

Synthetic Route B

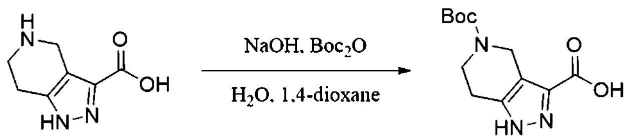


M3-1 M3-2

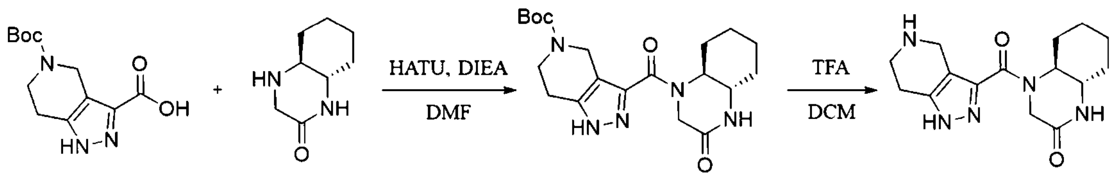


M3-2 M2 M3-3 II-12

Compound II-12 was obtained via Synthetic Route B.

11-12:

(4aS,8aS)-4-(4,5,6,7-tetrahydro-1H-pyrazolo[4,3-c]pyridine-3-carbonyl)octahydroquinoxalin-2(1H)-one

(1) At 0°C, a solution of 4,5,6,7-tetrahydro-2H-pyrazolo[4,3-c]pyridine-3-carboxylic acid (M3-1, 0.167 g, 1 mmol) in 2 mL of 1 mol/L NaOH and 0.6 mL of 1,4-dioxane was added dropwise with a solution of di-tert-butyl dicarbonate ($Boc_2O$, 0.25 g, 1.1 mmol) in 1.5 mL of 1,4-dioxane. The reaction mixture was stirred at 0°C for 0.5 h and then allowed to react overnight at room temperature, maintaining pH 8-9. After the reaction, the mixture was diluted with 5 mL of $H_2O$ and extracted with n-hexane. The aqueous phase was acidified to pH 2-3 with citric acid and extracted with ethyl acetate. The organic phase was washed with saturated brine, dried over anhydrous $MgSO_4$, and concentrated under reduced pressure to afford 5-(tert-butoxycarbonyl)-4,5,6,7-tetrahydro-2H-pyrazolo[4,3-c]pyridine-3-carboxylic acid (M3-2, 200 mg, 75%), LC-MS m/z = 268.1 [M+H]$^+$。

(2) M3-2 (20 mg, 0.075 mmol) was mixed with (4aS,8aS)-octahydroquinoline-2(1H)-one (M2, 15 mg, 0.1 mmol) and 2-(7-azabenzotriazol)-N,N,N',N'-tetramethyluronium hexafluorophosphate (HATU, 38 mg, 0.1 mmol), followed by addition of N,N-diisopropylethylamine (DIEA, 0.2 mL) and N,N-dimethylformamide (DMF, 2 mL). The mixture was stirred at room temperature for 18 hours. Ethyl acetate and water were added, and the organic layer was separated,

dried over sodium sulfate, and concentrated under reduced pressure. The residue was purified by silica gel column chromatography (ethyl acetate : hexane = 3:7) to give tert-butyl 3-((4aS,8aS)-3-oxodecahydroquinoline-1-carbonyl)-1,4,6,7-tetrahydro-5H-pyrazolo[4,3-c]pyridine-5-carboxylate (M3-3, 18 mg, 60%), LC-MS m/z = 404.2 $[M+H]^+$。
(3) M3-3 (18 mg, 0.04 mmol) and trifluoroacetic acid (TFA, 35 mg, 0.3 mmol) were added to 2 mL of dichloromethane (DCM) and stirred at room temperature for 30 minutes. Ethyl acetate and water were then added, and the organic layer was separated, dried over sodium sulfate, and concentrated under reduced pressure to afford II-12 (13 mg, 96%), LC-MS m/z = 304.2 $[M+H]^+$。

Synthetic Route C

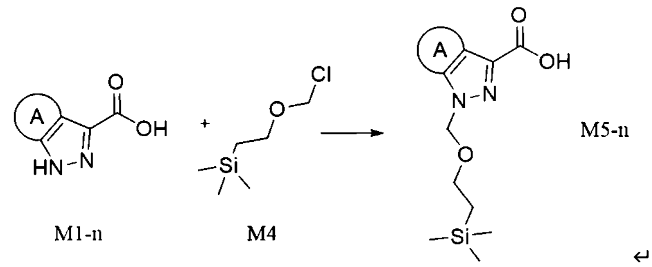

M1-n M4 M5-n

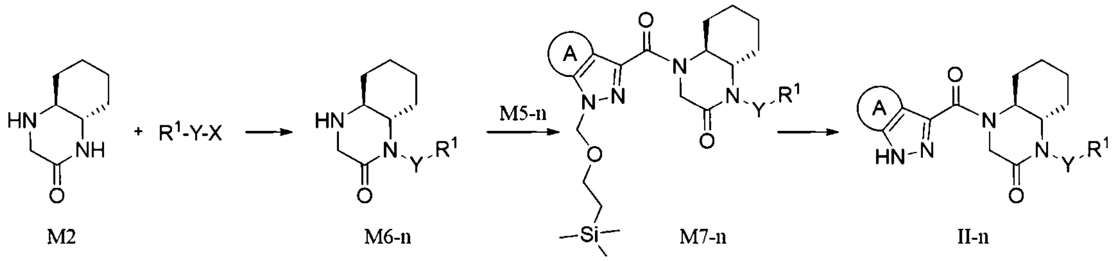


M2 M6-n M7-n II-n

(1) Sodium hydride (60% dispersion, 1.07 g, 26.9 mmol) dissolved in 5 mL of tetrahydrofuran (THF) was cooled to 0 °C. A solution of M1-n (21.5 mmol) in 50 mL of THF was added dropwise over 15 minutes, and the mixture was stirred at 0 °C for 1 hour. A solution of (2-(chloromethoxy)ethyl)trimethylsilane (M4, 4.76 mL, 26.9 mmol) in 50 mL of THF was then added dropwise, and the mixture was stirred at room temperature for 48 hours. Water and ethyl acetate were slowly added to the reaction mixture. The organic layer was separated, dried over sodium sulfate, and concentrated under reduced pressure to afford M5-n.
(2) In a 20 mL reaction vessel, $R^1$-Y-X (0.698 mmol, where X represents a halogen, and $R^1$ and Y are as defined above), (4aS,8aS)-octahydroquinoline-2(1H)-one (M2, 108 mg, 0.698 mmol, 1.0 eq.), cesium carbonate (796 mg, 2.443 mmol, 2.5 eq.), 4,5-bis(diphenylphosphino)-9,9-dimethylxanthene (20 mg, 0.035 mmol, 0.05 eq.) and palladium acetate (7.84 mg, 0.035 mmol, 0.05 eq.) were dissolved in 3 mL of THF and purged with nitrogen for 10 minutes. The reaction was heated to 70 °C and maintained for 90 minutes. The mixture was filtered, washed with dichloromethane, and concentrated. The residue was purified by silica gel column chromatography (0-10% ethyl acetate/dichloromethane) to afford M6-n.
(3) M6-n (1.0 mmol, 1.0 eq.) was dissolved in 10 mL of dichloromethane, followed by addition of M5-n (1.2 mmol, 1.2 eq.), triethylamine (200 mg, 2.0 mmol, 2.0 eq.) and 2-(7-azabenzotriazol)-N,N,N',N'-tetramethyluronium hexafluorophosphate (570 mg, 1.5 mmol, 1.5 eq.). The mixture was stirred overnight. The reaction was monitored by thin-layer chromatography, and upon disappearance of the starting material, the mixture was diluted with dichloromethane, washed with brine, dried over sodium sulfate, and concentrated under reduced pressure. The residue was purified by silica gel column chromatography (petroleum ether : ethyl acetate = 2:1) to afford M7-n.
(4) At 5 °C, triethylsilane (2.30 g, 19.8 mmol) was added to a solution of M7-n (3.95 mmol) in 39.5 mL of THF. The mixture was stirred at room temperature for 3 hours, concentrated, washed with brine, dried over sodium sulfate, and concentrated under reduced pressure. The residue was purified by silica gel column chromatography (ethyl acetate : petroleum ether = 20-100%) to afford the target product II-n.

Compounds II-2 to 11-10 were obtained via Synthetic Route C.

II-2:

(4aS,8aS)-1-(3-methylthiophen-2-yl)-4-(1,4,5,6-tetrahydrocyclopenta[c]pyrazole-3-carbonyl)octahydroquinoxalin-2(1H)-one (1504 mg, 99%), LC-MS m/z=384.2 $[M+H]^+$。

II-3 :

(4aS,8aS)-1-(2-methyloxazol-4-yl)-4-(1,4,5,6-tetrahydrocyclopenta[c]pyrazole-3-carbonyl)octahydroquinoxalin-2(1H)-one (1445 mg, 99%), LC-MS m/z=370.2 $[M+H]^+$。

II-4:

(4aS,8aS)-1-(3-methylisothiazol-5-yl)-4-(1,4,5,6-tetrahydrocyclopenta[c]pyrazole-3-carbonyl)octahydroquinoxalin-2(1H)-one (1507 mg, 99%), LC-MS m/z=386.2 $[M+H]^+$。

11-5:

(4aS,8aS)-1-(2-methylpyridin-4-yl)-4-(1,4,5,6-tetrahydrocyclopenta[c]pyrazole-3-carbonyl)octahydroquinoxalin-2(1H)-one (1469 mg, 98%), LC-MS m/z=380.2 $[M+H]^+$。

II-6 :

(4aS,8aS)-1-(6-methylpyridin-2-yl)-4-(1,4,5,6-tetrahydrocyclopenta[c]pyrazole-3-carbonyl)octahydroquinoxalin-2(1H)-one (1484 mg, 99%), LC-MS m/z=380.2 $[M+H]^+$。

II-7 :

(4aS,8aS)-1-(6-hydroxypyridin-2-yl)-4-(1,4,5,6-tetrahydrocyclopenta[c]pyrazole-3-carbonyl)octahydroquinoxalin-2(1H)-one (1491 mg, 99%), LC-MS m/z=382.2 $[M+H]^+$。

II-8:

(4aS,8aS)-1-(4-hydroxypyridin-2-yl)-4-(1,4,5,6-tetrahydrocyclopenta[c]pyrazole-3-carbonyl)octahydroquinoxalin-2(1H)-one (1491 mg, 99%), LC-MS m/z=382.2 $[M+H]^+$。

11-9:

(4aS,8aS)-1-(2-methylpyrimidin-4-yl)-4-(1,4,5,6-tetrahydrocyclopenta[c]pyrazole-3-carbonyl)octahydroquinoxalin-2(1H)-one (1488 mg, 99%), LC-MS m/z=381.2 $[M+H]^+$。

II-10:

(4aS,8aS)-1-(6-methylpyridazin-3-yl)-4-(1,4,5,6-tetrahydrocyclopenta[c]pyrazole-3-carbonyl)octahydroquinoxalin-2(1H)-one (1413 mg, 94%), LC-MS m/z=381.2 $[M+H]^+$。

Synthetic Route D

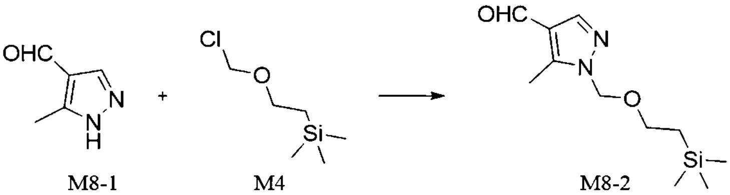

M8-1 M4 M8-2

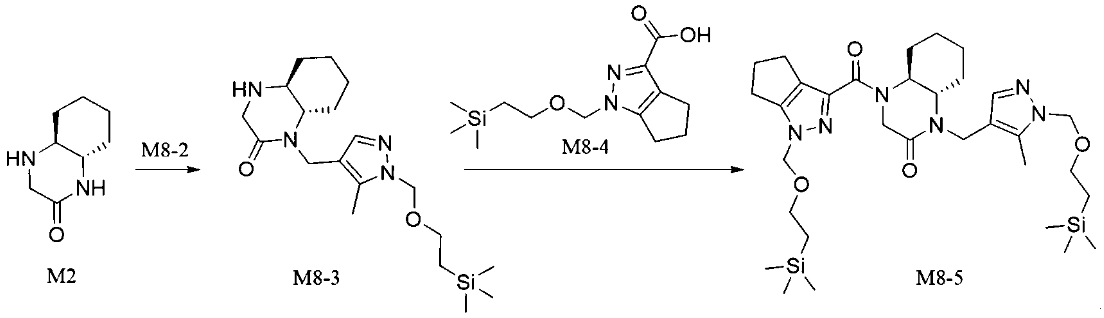


M2 M8-3 M8-5

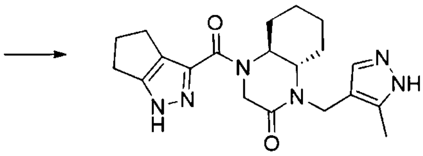

II-11

Compound II-11 was obtained via Synthetic Route D.

II-11:

(4aS,8aS)-1-((5-methyl-1H-pyrazol-4-yl)methyl)-4-(1,4,5,6-tetrahydrocyclopenta[c]pyrazole-3-carbonyl)octahydroquinoxalin-2(1H)-one

(1) Sodium hydride (60% dispersion in THF, 1.07 g, 26.9 mmol) dissolved in 5 mL of tetrahydrofuran (THF) was cooled to 0 °C. A solution of 5-methyl-1H-pyrazole-4-carbaldehyde (M8-1, 2.37 g, 21.5 mmol) in 50 mL of THF was added dropwise over 15 minutes, and the mixture was stirred at 0 °C for 1 hour. A solution of (2-(chloromethoxy)ethyl) trimethylsilane (M4, 4.76 mL, 26.9 mmol) in 50 mL of THF was added dropwise, and the mixture was stirred at room temperature for 48 hours. Water and ethyl acetate were slowly added, and the organic layer was separated, dried over sodium sulfate, and concentrated under reduced pressure to afford 5-methyl-1-((2-(trimethylsilyl)ethoxy)methyl)-1H-pyrazole-4-carbaldehyde (M8-2, 4.96 g, 96%). LC-MS m/z = 240.1 $[M+H]^+$。

(2) In a 20 mL reaction vessel, M8-2 (168 mg, 0.698 mmol), (4aS,8aS)-octahydroquinoline-2(1H)-one (M2, 108 mg, 0.698 mmol, 1.0 eq.), cesium carbonate (796 mg, 2.443 mmol, 2.5 eq.), 4,5-bis(diphenylphosphino)-9,9-dimethylxanthene (20 mg, 0.035 mmol, 0.05 eq.) and palladium acetate (7.84 mg, 0.035 mmol, 0.05 eq.) were dissolved in 3 mL of THF and purged with nitrogen for 10 minutes. The reaction was heated to 70 °C and maintained for 90 minutes. The mixture was filtered, washed with dichloromethane, and concentrated. The residue was purified by silica gel column chromatography (0-10% ethyl acetate/dichloromethane) to give (4aS,8aS)-1-((5-methyl-1-((2-(trimethylsilyl)ethoxy) methyl)-1H-pyrazol-4-yl)methyl)octahydroquinoline-2(1H)-one (M8-3, 244 mg, 92.2%). LC-MS m/z = 378.3 $[M+H]^+$。

(3) M8-3 (379 mg, 1.0 mmol, 1.0 eq.) was dissolved in 10 mL of dichloromethane, followed by addition of 1-((2-(trimethylsilyl)ethoxy)methyl)-1,4,5,6-tetrahydrocyclopenta[c]pyrazole-3-carboxylic acid (M8-4, 339 mg, 1.2 mmol, 1.2 eq.), triethylamine (200 mg, 2.0 mmol, 2.0 eq.) and 2-(7-azabenzotriazol)-N,N,N',N'-tetramethyluronium hexafluorophosphate (570 mg, 1.5 mmol, 1.5 eq.). The mixture was stirred overnight. The reaction was monitored by thin-layer chromatography, and upon disappearance of the starting material, the mixture was diluted with dichloromethane, washed with brine, dried over sodium sulfate, and concentrated under reduced pressure. The residue was purified by silica gel column chromatography (petroleum ether:ethyl acetate = 2:1) to afford (4aS,8aS)-1-((5-methyl-1-((2-(trimethylsilyl)ethoxy)methyl)-1H-pyrazol-4-yl)methyl)-4-(1-((2-(trimethylsilyl)ethoxy)methyl)-1,4,5,6-tetrahydrocyclopenta[c]pyrazole-3-carbonyl)octahydroquinoxalin-2(1H)-one (M8-5, 424 mg, 66%). LC-MS m/z = 642.4 $[M+H]^+$。

(4) At 5 °C, triethylsilane (2.30 g, 19.8 mmol) was added to a solution of M8-5 (2540 mg, 3.95 mmol) in 39.5 mL of THF. The mixture was stirred at room temperature for 3 hours, concentrated, washed with brine, dried over sodium sulfate,

and concentrated under reduced pressure. The residue was purified by silica gel column chromatography (ethyl acetate : petroleum ether = 20-100%) to afford 11-11 (1510 mg, 99%). LC-MS m/z = 383.2 $[M+H]^+$.

Example 1

Molecular docking of the pyrazolecarbonyl piperazinone compounds of the present application with the BMPR1B target protein was performed using AutoDock Vina software, generating 13 docking conformations for each compound. The binding energy of the optimal docking conformation between each compound and BMPR1B was calculated. The docking results are shown in Table 1. Column 2 of Table 1 represents the binding free energy calculated by AutoDock Vina; the more negative the value, the stronger the compound binds to the target protein. Predicted values show that the absolute values of the binding energies of the pyrazolecarbonyl piperazinone compounds are all significantly greater than the threshold value of 3 set according to the target features.

Cytotoxicity prediction for the pyrazolecarbonyl piperazinone compounds was performed using eToxPred software. The cytotoxicity results for each compound are shown in Column 3 of Table 1. Predicted values indicate that all compounds have toxicity below the threshold of 0.58 (values above 0.58 suggest potential toxicity).

Table 1. Molecular Docking Binding Energies and Predicted Cytotoxicity of Pyrazolecarbonyl Piperazinone Compounds

| Compound Number | Molecular Docking Binding Energy (kcal/mol) | Predicted Cytotoxicity |
|---|---|---|
| II-1 | -9.5 | 0.54 |
| II-2 | -9.9 | 0.48 |
| II-3 | -10.6 | 0.50 |
| II-4 | -10.1 | 0.48 |
| II-5 | -11.8 | 0.49 |
| II-6 | -11.7 | 0.48 |
| II-7 | -12 | 0.47 |
| II-8 | -11.9 | 0.52 |
| II-9 | -11.4 | 0.46 |
| II-10 | -11.7 | 0.49 |
| II-11 | -10.2 | 0.47 |
| II-12 | -8.9 | 0.52 |
| II-13 | -10.2 | 0.57 |

Example 2

A total of 330 kinase candidates (15-50 nM, 2.5 μL) were mixed with compound II-1 (2.5 mM, 25 nL, prepared in DMSO) and incubated at 25 °C for 10 minutes. Then, a mixture of kinase peptide substrate (0.2 mg/mL; supplier: GenScript) and ATP (10-60 μM; supplier: Promega; product number: V915B) was added (total 2.5 μL, prepared in assay buffer), and the reaction was carried out at 25 °C for 60-120 minutes. Finally, the reaction products were quantified using HTRF1 or ADP-Glo2 methods. During the kinase activity assay, the consumption of each substrate was kept below 10%, and each kinase assay was performed in duplicate.

The results showed that II-1 exhibited significant inhibitory activity against BMPR1B kinase (Figure 1A; note: all test results are displayed, but for clarity, only one kinase name is shown on the x-axis for every 10 kinases). At the same concentration, II-1 did not inhibit the kinase activity of TGFBR1 or BMPR2. Furthermore, at different concentrations (up to 12.5 μM, threefold serial dilution), II-1 still showed no inhibition of TGFBR1 or BMPR2 kinase (Figure 1B, C). These results confirm the selective binding ability of II -1 to its target.

To examine the above results from a structural perspective, PyMol software (supplier: Schrödinger) was used to analyze the complexes of compounds II-1, II-2, II-11, and II-13 with BMPR1B. As shown in Figure 2, the complex structures were obtained by molecular docking. The N- and C-terminal domains of BMPR1B are represented as cartoon models of different shapes. The loop structures of BMPR1B, including the activation loop, are represented as cartoon models with different shades of gray. The pyrazolecarbonyl piperazinone compounds are shown as stick-and-ring models, the interacting amino acid backbones/side chains are shown as stick models, hydrogen bonds are represented as solid lines, and hydrophobic interactions are represented as dashed lines. These results also indicate that pyrazolecarbonyl piperazinone compounds have structural features consistent with binding to BMPR1B.

Example 3

Tumor cells OVCAR3 (ovarian cancer), HeLa (breast cancer), U251 (glioma), H9 cells, and non-tumor HEK293 cells were cultured in DMEM/F12 medium supplemented with 10% FBS and 1% P/S. When cells reached 70% confluency, they were digested with 0.25% Trypsin at 37°C for 2-3 minutes, and digestion was stopped with DMEM/F12 medium. Cell suspensions were collected in 15 mL centrifuge tubes, centrifuged at 1000 rpm for 5 minutes, the supernatant was discarded, and cells were resuspended in DMEM/F12 medium with 10% FBS and 1% P/S. Cells were counted using a hemocytometer, and based on the counts, they were resuspended to the desired concentration. Tumor cells were seeded in 96-well plates at 1×10^4 cells per well, 100 μL per well. From day 1 to day 7 after seeding, cells were treated with compound II-1 at 0 μM, 10 μM, 30 μM, and 50 μM (the pyrazolecarbonyl piperazinone compounds were diluted to 10 μM, 30 μM, and 50 μM with DMEM + 10% FBS). Each concentration was tested in triplicate wells.

Cell viability on days 1, 3, 5, and 7 was determined using the CCK-8 cell proliferation/cytotoxicity assay kit (Vazyme, A311-02) by measuring absorbance at OD450 nm, with wells containing only medium and CCK-8 solution as blank controls. Each concentration was measured in triplicate. Based on the absorbance values, cell survival rates at different time points were calculated, as shown in Table 2.

Calculation of cell survival rate: For the same cell type at the same time point, the absorbance of cells not treated with compound II-1 was set as 100% baseline. For cells treated with different concentrations, the absorbance was compared with the control group (CK) to calculate the relative proportion. For example, the survival rate of H9 cells on day 4 treated with 30 μM of the small molecule compound was calculated as follows:

$$[\mathrm{OD}（\mathrm{D4}）30\ \mu\mathrm{M\ iPS}]/[\mathrm{OD}（\mathrm{D4}）0\ \mu\mathrm{M\ H9}]\times 100\%$$

Table 2. Cell survival rates of various cell types after treatment with different concentrations of compound II-1 measured by CCK-8 assay

| Cell Type | OVCAR3 | | | Hela | | | U251 | | |
|---|---|---|---|---|---|---|---|---|---|
| II-1 Concentration | 10 μM | 30 μM | 50 μM | 10 μM | 30 μM | 50 μM | 10 μM | 30 μM | 50 μM |
| Day 1 Treatment | 95.05% | 89.67% | 88.56% | 103.32% | 100.64% | 88.66% | 100.75% | 99.49% | 98.61% |
| Day 3 Treatment | 98.06% | 94.18% | 90.37% | 98.58% | 93.45% | 81.80% | 98.96% | 87.01% | 85.27% |
| Day 5 Treatment | 96.19% | 92.35% | 85.37% | 97.35% | 90.71% | 80.31% | 95.19% | 79.39% | 70.43% |
| Day 7 Treatment | 97.99% | 90.98% | 80.83% | 97.27% | 85.04% | 75.83% | 94.74% | 74.00% | 63.89% |

| Cell Type | H9 | | | HEK293 | | |
|---|---|---|---|---|---|---|
| II-1 Concentration | 10 μM | 30 μM | 50 μM | 10 μM | 30 μM | 50 μM |
| Day 1 Treatment | 90.37% | 84.96% | 76.96% | 96.09% | 93.69% | 80.80% |
| Day 3 Treatment | 92.91% | 86.01% | 73.87% | 96.67% | 94.14% | 90.72% |
| Day 5 Treatment | 84.84% | 80.25% | 69.10% | 98.80% | 94.39% | 92.46% |
| Day 7 Treatment | 83.47% | 78.71% | 65.98% | 99.24% | 94.97% | 93.56% |

The results showed that, for the three tumor cell lines Hela, U251, and OVCAR3, treatment with the same concentration of small molecule compound II-1 led to a gradual decrease in cell viability over time. Additionally, as the concentration of compound II-1 increased, cell viability also decreased. Among them, U251 cells were more sensitive to the small molecule compound, exhibiting lower cell viability compared to Hela and OVCAR3 cells.

As an atypical representative of embryonic tumors (such as germ cell tumors), H9 cells also showed a gradual decline in viability with increasing compound II-1 concentration and prolonged treatment time. However, for non-tumor HEK293 cells, cell viability did not show significant changes with increasing treatment time or compound II-1 concentration, indicating that compound II-1 exhibits more specific cytotoxicity toward tumor cells.

Example 3

In this example, compound II-1 and the reference compound LY2157299 were used to treat glioma U251 cells.

Samples were collected at 12 hours and 72 hours posttreatment. Total RNA was extracted using the Rneasy Mini or Micro Kit (QIAGEN), and 1 mg of RNA was reverse-transcribed into cDNA using the SuperScript III First-Strand Synthesis System (Invitrogen). Quantitative PCR was performed using SYBR Premix Ex Taq (TaKaRa) on a Thermal Cycler Dice Real-Time System (TaKaRa), with beta-Actin serving as an internal control. All data were analyzed using the delta-Ct method. Each experiment was performed in triplicate, and variance analysis was conducted. Primer sequences for genes encoding different cell markers are listed in Table 3. The results, shown in Figure 3, demonstrated that at the same concentration, compound II-1 induced higher apoptosis compared to the anti-tumor compound LY2157299, and after 72 hours of treatment, fewer cells remained in the wells treated with compound II-1. These results indicate that, compared with the reference compound LY2157299, the pyrazolecarbonyl piperazinone compounds of the present invention exhibit stronger cytotoxic effects on tumor cells.

Table 3. Primers for transcriptional analysis of cells treated with compound II-1

| Gene | Primer Sequence | SEQ ID NO. |
|---|---|---|
| CASP3-F | GCTGCCTGTAACTTGAGAGTAGAT | SEQ ID NO:1 |
| CASP3-R | CCTTCCTGCGTGGTCCAT | SEQ ID NO:2 |
| ACTIN-F | TCCCTGGAGAAGAGCTACGA | SEQ ID NO:3 |
| ACTIN-R | AGCACTGTGTTGGCGTACAG | SEQ ID NO:4 |

The technical features described in the above examples can be combined in any manner. For the sake of brevity, not all possible combinations of the technical features in the above examples have been explicitly described. However, as long as the combination of these technical features does not result in a contradiction, it should be regarded as falling within the scope disclosed in this specification.

The above examples merely illustrate several embodiments of the present application in a specific and detailed manner, and should not be construed as limiting the scope of the invention. It should be noted that those skilled in the art, within the framework of the present application, may make various modifications and improvements, all of which are considered to fall within the protection scope of the present application. Therefore, the protection scope of the present patent application shall be defined by the appended claims, with the description and drawings serving to explain the content of the claims.

## Claims

1. A pyrazolecarbonyl piperazinone compound, wherein the pyrazolecarbonyl piperazinone compound has a structure represented by Formula I, or a pharmaceutically acceptable salt, ester, amide, solvate, active metabolite, polymorph, isotopically labeled compound, isomer, or prodrug thereof;

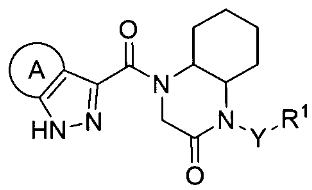

I

wherein the ring A is selected from any one of the following structures:

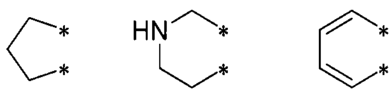

Y is $-(CH_2)_n-$, where n is 0, 1, 2, or 3;
$R^1$ is selected from -H, -D, unsubstituted or $R^2$-substituted $C_1$-$C_6$ alkyl, $C_1$-$C_6$ alkoxy, $C_6$-$C_{10}$ aryl, or $C_3$-$C_{10}$ heteroaryl;
$R^2$ is selected from -F, -Cl, -Br, -I, -OH, -COOH, $C_1$-$C_6$ alkyl, or $C_1$-$C_6$ alkoxy;
"*" represents a point of attachment.

**2.** The pyrazolecarbonyl piperazinone compound according to claim 1, wherein the pyrazolecarbonyl piperazinone compound satisfies one or more of the following conditions:

(1) Y is $-(CH_2)_n-$, where n is 0, 1, or 2;
(2) $R^1$ is selected from -H, -D, unsubstituted or $R^2$-substituted $C_1$-$C_6$ alkyl, $C_6$-$C_{10}$ aryl, or $C_3$-$C_{10}$ heteroaryl;
(3) $R^2$ is selected from -OH or $C_1$-$C_6$ alkyl.

**3.** The pyrazolecarbonyl piperazinone compound according to claim 2, wherein the pyrazolecarbonyl piperazinone compound satisfies one or more of the following conditions:

(1) Y is $-(CH_2)_n-$, where n is 0 or 1;
(2) $R^1$ is selected from -H, -D, unsubstituted or $R^2$-substituted $C_3$-$C_6$ heteroaryl;
(3) $R^2$ is selected from -OH or $C_1$-$C_4$ alkyl.

**4.** The pyrazolecarbonyl piperazinone compound according to claim 3, wherein the pyrazolecarbonyl piperazinone compound satisfies one or more of the following conditions:

(1) Y is $-(CH_2)_n-$, where n is 0 or 1;
(2) $R^1$ is selected from -H, -D, unsubstituted or $R^2$-substituted furyl, thienyl, pyrrolyl, pyrazolyl, imidazolyl, oxazolyl, isoxazolyl, thiazolyl, isothiazolyl, pyranyl, thianyl, pyridyl, diazinyl, or pyrimidinyl;
(3) $R^2$ is selected from -OH, methyl, ethyl, n-propyl, or isopropyl.

**5.** The pyrazolecarbonyl piperazinone compound according to any one of claims 1 to 4, wherein $R^1$ is selected from -H, -D, or any one of the following substituents:

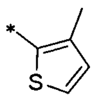 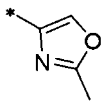 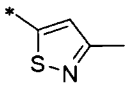 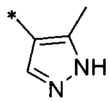 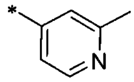

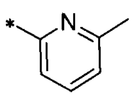 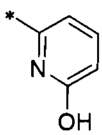 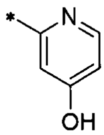 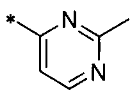 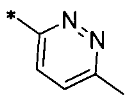

wherein "*" represents a point of attachment.

**6.** The pyrazolecarbonyl piperazinone compound according to any one of claims 1 to 4, wherein the pyrazolecarbonyl piperazinone compound has a structure represented by any one of Formulas I-1 to I-13, or a pharmaceutically acceptable salt, ester, amide, solvate, active metabolite, polymorph, isotopically labeled compound, isomer, or prodrug thereof;

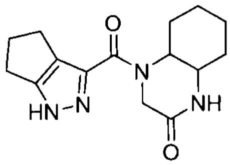
I-1

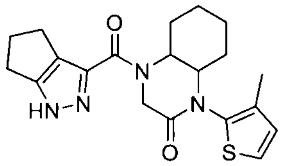
I-2

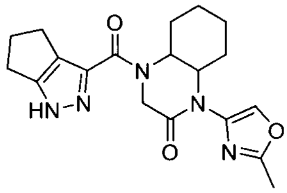
I-3

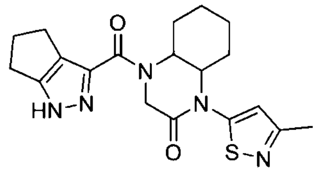
I-4

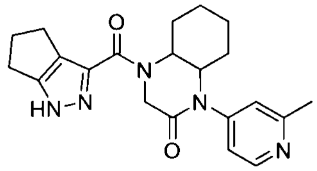
I-5

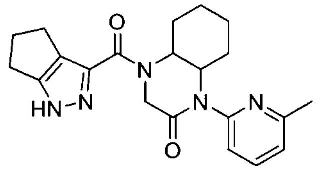
I-6

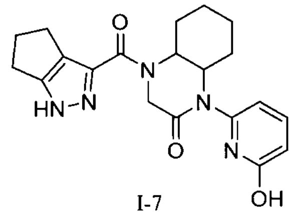
I-7

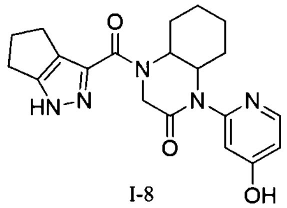
I-8

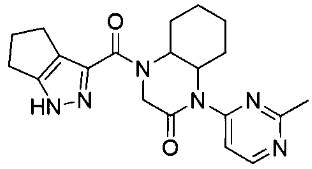
I-9

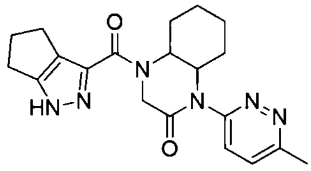
I-10

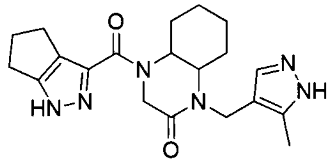
I-11

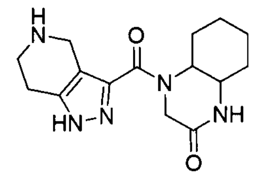
I-12

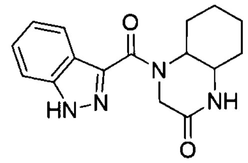
I-13

**7.** The pyrazolecarbonyl piperazinone compound according to any one of claims 1 to 4, wherein the pyrazolecarbonyl piperazinone compound has a structure represented by Formula II, or a pharmaceutically acceptable salt, ester, amide, solvate, active metabolite, polymorph, isotopically labeled compound, isomer, or prodrug thereof;

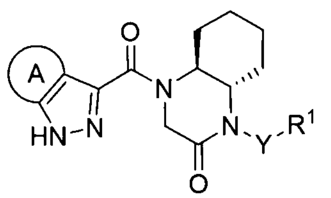
II

wherein ring A, Y, and $R^1$ are as defined in any one of claims 1 to 4.

**8.** The pyrazolecarbonyl piperazinone compound according to any one of claims 1 to 4, wherein the pyrazolecarbonyl piperazinone compound has a structure represented by any one of Formulas II-1 to II-13, or a pharmaceutically acceptable salt, ester, amide, solvate, active metabolite, polymorph, isotopically labeled compound, isomer, or prodrug thereof;

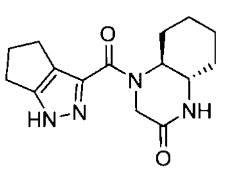
II-1

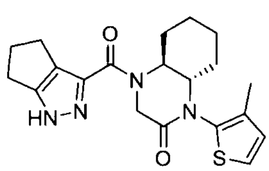
II-2

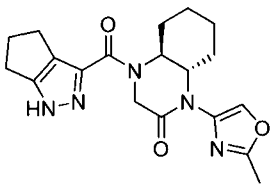
II-3

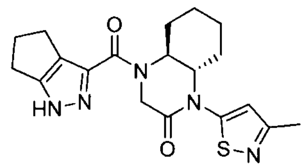
II-4

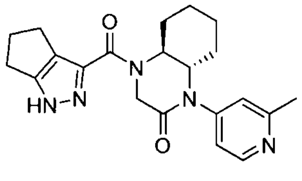
II-5

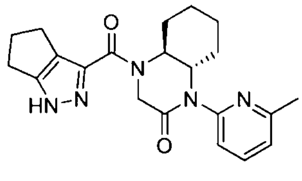
II-6

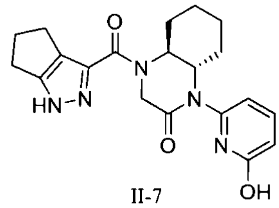
II-7

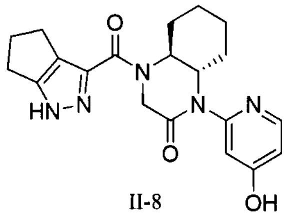
II-8

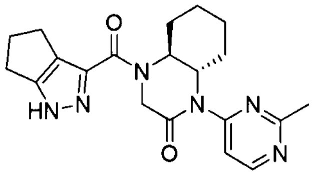
II-9

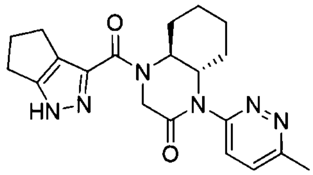
II-10

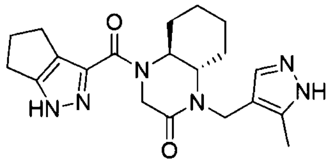
II-11

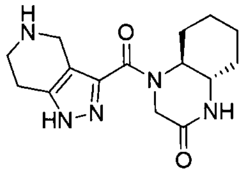
II-12

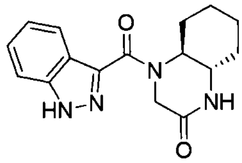
II-13

.

**9.** A pharmaceutical composition comprising the pyrazolecarbonyl piperazinone compound according to any one of claims 1 to 8, and at least one pharmaceutically acceptable carrier.

**10.** Use of the pyrazolecarbonyl piperazinone compound according to any one of claims 1 to 8, or the pharmaceutical composition according to claim 9, in the preparation of a medicament for treating one or more of the following diseases: cancer and pulmonary arterial hypertension.

**11.** The use according to claim 10, wherein the cancer is one or more selected from ovarian cancer, breast cancer, glioma, and germ cell tumor.

**12.** Use of the pyrazolecarbonyl piperazinone compound according to any one of claims 1 to 8 as a BMPR1B inhibitor.

**13.** The use according to claim 12, wherein the concentration of the pyrazolecarbonyl piperazinone compound is 10 $\mu$M to 100 $\mu$M.

**14.** A method for inhibiting BMPR1B expression, comprising the step of contacting the pyrazolecarbonyl piperazinone compound according to any one of claims 1 to 8 with cells, biological tissues, or organoids comprising BMPR1B.

**15.** The method according to claim 14, wherein the concentration of the pyrazolecarbonyl piperazinone compound during the contacting step is 10 $\mu$M to 100 $\mu$M.

**16.** A method for treating cancer or pulmonary arterial hypertension, comprising the step of administering to a subject a therapeutically effective amount of the pyrazolecarbonyl piperazinone compound according to any one of claims 1 to 8, or administering to a subject a therapeutically effective amount of the pharmaceutical composition according to claim 9.

**17.** The method according to claim 16, wherein the cancer is one or more selected from ovarian cancer, breast cancer, and glioma.

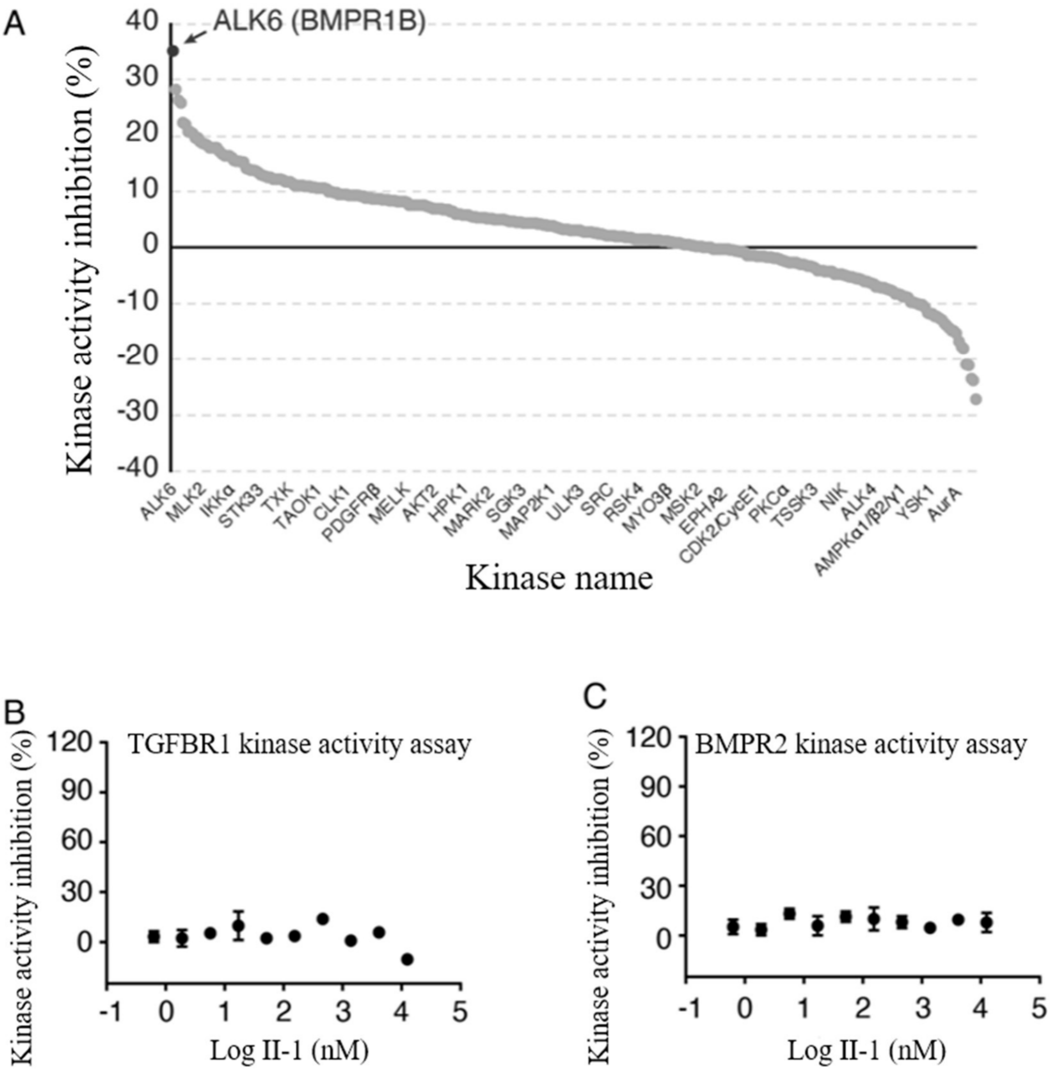
A
ALK6 (BMPR1B)
Kinase activity inhibition (%)
40
30
20
10
0
-10
-20
-30
-40
ALK6
MLK2
IKKα
STK33
TXK
TAOK1
CLK1
PDGFRβ
MELK
AKT2
HPK1
MARK2
SGK3
MAP2K1
ULK3
SRC
RSK4
MYO3β
MSK2
EPHA2
CDK2/CycE1
PKCα
TSSK3
NIK
ALK4
AMPKα1/β2/γ1
YSK1
AurA
Kinase name
B
TGFBR1 kinase activity assay
Kinase activity inhibition (%)
120
90
60
30
0
-1
0
1
2
3
4
5
Log II-1 (nM)
C
BMPR2 kinase activity assay
Kinase activity inhibition (%)
120
90
60
30
0
-1
0
1
2
3
4
5
Log II-1 (nM)

FIG. 1

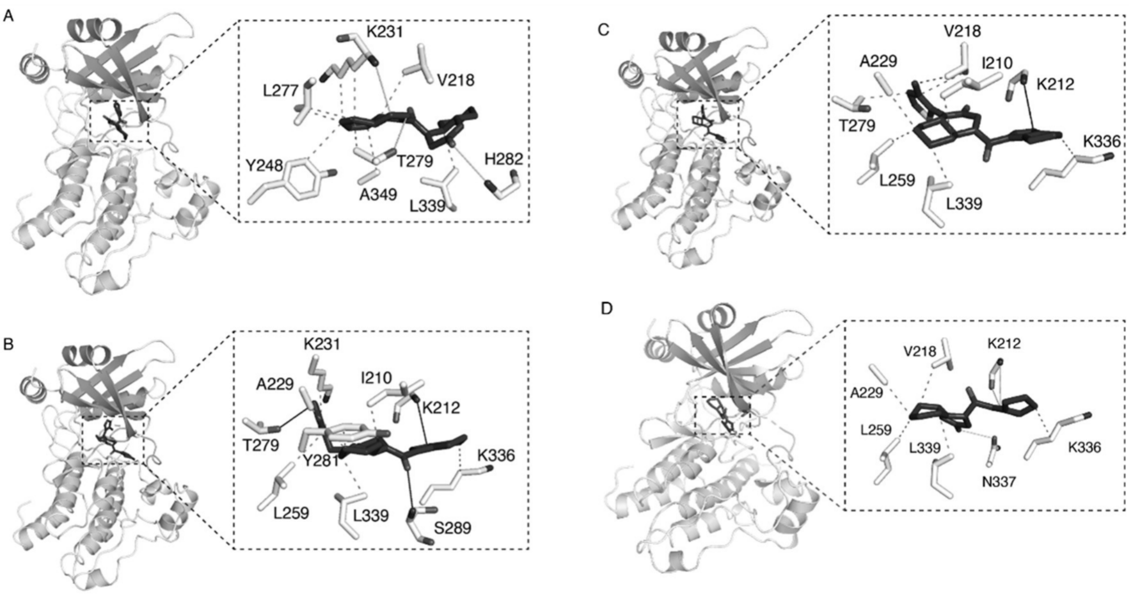
A
K231
L277
V218
Y248
T279
H282
A349
L339
B
K231
A229
I210
K212
T279
Y281
K336
L259
L339
S289
C
V218
A229
I210
K212
T279
K336
L259
L339
D
V218
K212
A229
L259
L339
K336
N337

FIG. 2

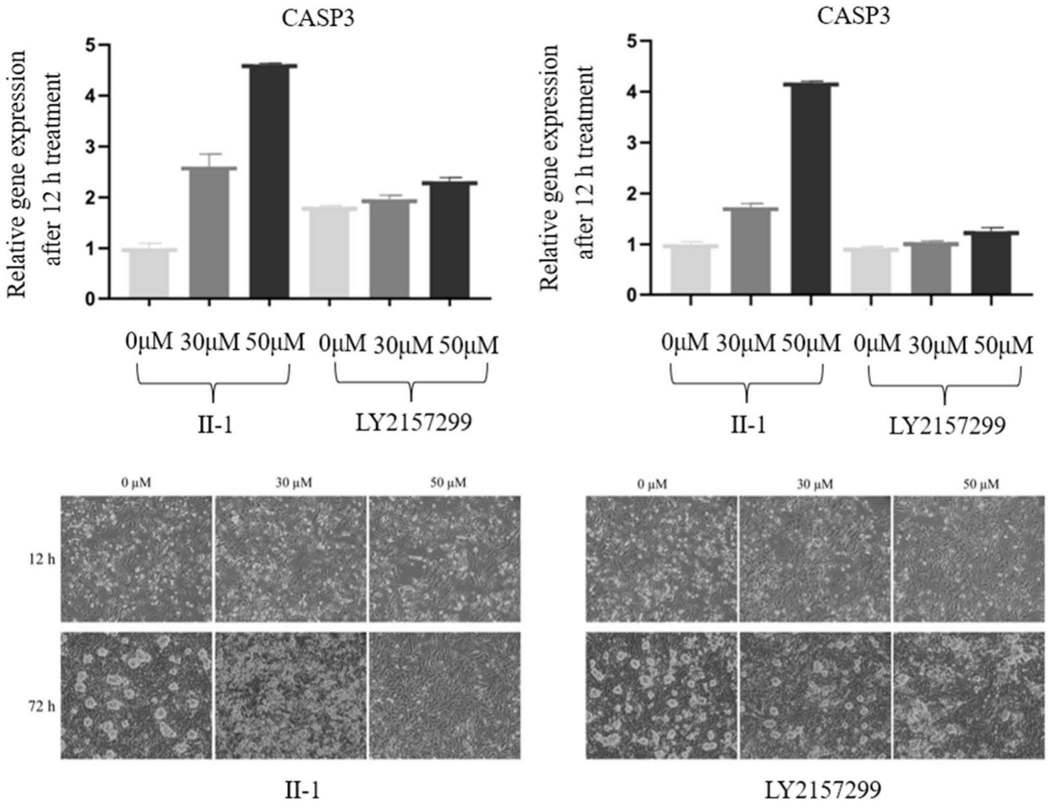
CASP3
Relative gene expression after 12 h treatment
0μM 30μM 50μM 0μM 30μM 50μM
II-1
LY2157299
CASP3
Relative gene expression after 12 h treatment
0μM 30μM 50μM 0μM 30μM 50μM
II-1
LY2157299
0 μM
30 μM
50 μM
12 h
72 h
II-1
0 μM
30 μM
50 μM
LY2157299

FIG. 3

## INTERNATIONAL SEARCH REPORT

International application No.
**PCT/CN2023/095443**

**A. CLASSIFICATION OF SUBJECT MATTER**

C07D 403/06(2006.01)i

According to International Patent Classification (IPC) or to both national classification and IPC

**B. FIELDS SEARCHED**

Minimum documentation searched (classification system followed by classification symbols)

IPC:C07D

Documentation searched other than minimum documentation to the extent that such documents are included in the fields searched

Electronic data base consulted during the international search (name of data base and, where practicable, search terms used)

CNTXT, CNABS, DWPI, ENTXTC, VCN, WPABS, STN, CNKI, ISI-Web of Science: 睿健医药, 吡唑甲酰, 哌嗪, 抑制剂, 结构式I, structural formula I, BMPR1B, TGF, transforming growth factor, ALK, +pyrazole+, +piperazine+, inhibitor

**C. DOCUMENTS CONSIDERED TO BE RELEVANT**

| Category* | Citation of document, with indication, where appropriate, of the relevant passages | Relevant to claim No. |
|---|---|---|
| X | STN Registry. "STN Registry"<br>*STN Registry*, 29 September 2015 (2015-09-29),<br>pp. 2-3 | 1-8 |
| A | STN Registry. "STN Registry"<br>*STN Registry*, 29 September 2015 (2015-09-29),<br>pp. 2-3 | 9-13 |
| A | CN 113347965 A (FUNDACIO INSTITUT DE RECERCA BIOMEDICA (IRB BARCELONA) et al.) 03 September 2021 (2021-09-03)<br>claims 1-7 | 1-13 |
| A | US 2020239462 A1 (CLAVIUS PHARMACEUTICALS LLC) 30 July 2020 (2020-07-30)<br>entire document | 1-13 |
| A | CN 107406412 A (RIGEL PHARMACEUTICALS, INC. et al.) 28 November 2017 (2017-11-28)<br>entire document | 1-13 |

☑ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 November 2023** | **05 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/095443**

**C. DOCUMENTS CONSIDERED TO BE RELEVANT**

| Category* | Citation of document, with indication, where appropriate, of the relevant passages | Relevant to claim No. |
|---|---|---|
| A | CN 1694708 A (SCIOS INC.) 09 November 2005 (2005-11-09)<br>entire document | 1-13 |

Form PCT/ISA/210 (second sheet) (July 2022)

**INTERNATIONAL SEARCH REPORT**

International application No.
**PCT/CN2023/095443**

**Box No. I  Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/095443**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-17**
because they relate to subject matter not required to be searched by this Authority, namely:

Claims 14 and 15 set forth a method for inhibiting the expression of BMPR1B, and claims 16 and 17 set forth a method for treating cancer or pulmonary arterial hypertension, which methods fall within the cases set out in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/095443**

| Patent document cited in search report | Publication date (day/month/year) | Patent family member(s) | Publication date (day/month/year) |
|---|---|---|---|
| CN 113347965 A | 03 September 2021 | WO 2020104648 A2 | 28 May 2020 |
| | | WO 2020104648 A3 | 02 July 2020 |
| | | CA 3118796 A1 | 28 May 2020 |
| | | JP 2022517484 A | 09 March 2022 |
| | | US 2022089600 A1 | 24 March 2022 |
| | | EP 3883556 A2 | 29 September 2021 |
| US 2020239462 A1 | 30 July 2020 | JP 2020512400 A | 23 April 2020 |
| | | EP 3600294 A1 | 05 February 2020 |
| | | EP 3600294 A4 | 26 August 2020 |
| | | US 2022073514 A1 | 10 March 2022 |
| | | WO 2019005241 A1 | 03 January 2019 |
| | | US 11124509 B2 | 21 September 2021 |
| | | JP 2023052027 A | 11 April 2023 |
| CN 107406412 A | 28 November 2017 | AR 103828 A1 | 07 June 2017 |
| | | SG 11201705821 XA | 30 August 2017 |
| | | WO 2016140884 A1 | 09 September 2016 |
| | | PH 12017501326 A1 | 18 December 2017 |
| | | KR 20170122799 A | 06 November 2017 |
| | | AU 2016226468 A1 | 03 August 2017 |
| | | AU 2016226468 B2 | 22 October 2020 |
| | | CA 2973602 A1 | 09 September 2016 |
| | | EP 3265454 A1 | 10 January 2018 |
| | | EP 3265454 B1 | 26 February 2020 |
| | | ES 2789331 T3 | 26 October 2020 |
| | | MX 2017010735 A | 04 December 2017 |
| | | MX 371167 B | 21 January 2020 |
| | | MY 186133 A | 24 June 2021 |
| | | EA 037112 B1 | 08 February 2021 |
| | | US 2016257690 A1 | 08 September 2016 |
| | | US 9884868 B2 | 06 February 2018 |
| | | HK 1244785 A1 | 17 August 2018 |
| | | BR 112017017135 A2 | 03 April 2018 |
| | | TW 201639827 A | 16 November 2016 |
| | | JP 2018510857 A | 19 April 2018 |
| | | JP 6836998 B2 | 03 March 2021 |
| | | CN 107406412 B | 16 July 2021 |
| | | VN 56872 A | 26 April 2018 |
| | | ID 201804882 A | 11 May 2018 |
| | | IN 201717032477 A | 22 December 2017 |
| | | HK 1244785 A0 | 17 August 2018 |
| | | ZA 201705527 A | 28 August 2019 |
| CN 1694708 A | 09 November 2005 | BR 0314196 A | 26 July 2005 |
| | | AU 2003272324 A1 | 30 April 2004 |
| | | US 2004132730 A1 | 08 July 2004 |
| | | WO 2004024159 A1 | 25 March 2004 |
| | | JP 2006503043 A | 26 January 2006 |
| | | EP 1549316 A1 | 06 July 2005 |
| | | EP 1549316 A4 | 09 April 2008 |
| | | IN 200500525 P2 | 24 June 2006 |
| | | SG 110833 B | 30 March 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WAKEFIELD LM** ; **HILL CS**. *Nat Rev Cancer*, 2013, vol. 13, 328-341
- **MASSAGUÉ. J.** *Cell.*, 2008, vol. 134, 215-230
- **RODON J et al.** *Clin Cancer Res*, 2015, vol. 21, 553-560
- **MELISI D et al.** *Cancer Res*, 2016, vol. 76 (14), CT068-CT068